# FASCICULE DE BREVET EUROPEEN

(11) **EP 0 593 314 B1**
(45) Date de publication et mention de la délivrance du brevet: **03.07.1996**
(21) Numéro de dépôt: 93400882.2
(22) Date de dépôt: 05.04.1993
(51) Int. Cl.: C07C 319/28, C07C 323/52

(54) **Procédé de désodorisation d'un mercaptoacide et produit désodorisé ainsi obtenu**
Verfahren zur Deodorisierung von einer Mercaptosäure sowie damit erhältliches Produkt
Procedure to deodorise a mercapto acid and product thus obtained

(30) Priorité: 15.10.1992 FR 9212344
(43) Date de publication de la demande: 20.04.1994
(73) Titulaire: L'OREAL, F-75008 Paris (FR)
(72) Inventeur: Dedieu, Michel, F-78600 Maisons Laffitte (FR); Burgaud, Hervé, F-77230 Dammartin en Goele (FR); Lapoirie, Eric, F-93250 Villemomble (FR); Gurfein, Véronique 18, rue des Sources, FR - 92350 Le Plessis Robinson (FR); Malle, Gerard, F-77580 Villiers s/Morin (FR)
(74) Mandataire: Peuscet, Jacques

(56) Documents cités:
- PATENT ABSTRACTS OF JAPAN vol. 8, no. 114 (C-225)26 Mai 1984& JP-A-57 027 866 RAION) 14 Février 1984
- ANGEWANDTE CHEMIE, INTERNATIONAL EDITION IN ENGLISH, vol. 17, no. 10, Octobre 1978, Weinheim, DE, pages 710 - 715 P. HUBERT, ET AL.: 'The processing of some natural products with carbon dioxide' *
- FOOD TECHNOLOGY, vol. 42, no. 6, Juin 1988, Chicago, US, pages 145 - 150 F. TEMELLI, ET AL.: 'Supercritical fluid extraction in citrus oil processing' *
- COSMETICS AND TOILETRIES, vol. 106, no. 8, Août 1991, Oak Park, Ill, US, pages 61 - 67 J.W. KING: 'Supercritical fluid processing of cosmetic raw materials' *

## Description

La présente invention concerne un procédé de désodorisation d'un mercaptoacide et le produit désodorisé ainsi obtenu.

Les mercaptoacides sont des dérivés bien connus, qui ont une fonction thiol (-SH) et une fonction acide (- COOH). Ils ont de nombreuses applications. L'acide thioglycolique (ou mercaptoacétique), par exemple, peut être utilisé, sous sa forme acide, comme intermédiaire dans la synthèse de nombreux pesticides et produits pharmaceutiques, ou, sous sa forme acide ou salifiée (en particulier, sel d'ammonium, d'amine, de sodium, de potassium ou de calcium), pour le décapage de surfaces métalliques, le traitement de minerais sulfurés et le traitement des cuirs et peaux ; dans l'industrie cosmétique, il constitue également l'agent réducteur le plus utilise pour la déformation permanente des cheveux (frisage ou défrisage) et la matière active principale des laits et crèmes dépilatoires. De même, l'acide thiolactique (acide 2-mercaptopropionique) est utilisé comme réducteur pour la déformation permanente des cheveux ou comme constituant des laits et crèmes dépilatoires.

Les mercaptoacides purs ont une légère odeur piquante qui n'est pas vraiment déplaisante. Mais ils contiennent toujours, en pratique, des composés sulfurés, tels que l'hydrogène sulfuré et des mercaptans de faible poids moléculaire, en particulier le méthanethiol ou l'éthanethiol, qui ont une odeur nauséabonde particulièrement désagréable. Il suffit de très faibles quantités de ces composés sulfurés pour que l'on perçoive leur présence à l'odorat, le nez étant dans ce cas le meilleur instrument de détection.

La présence de ces composés malodorants est liée à divers processus de décomposition des mercaptoacides, processus qui sont encore très mal connus mais sont, sans doute, dus à la fois à des mécanismes ioniques et radicalaires pouvant se faire à l'abri de l'air. Cette décomposition et la formation de composés malodorants, qui en résulte, peuvent, d'ailleurs, être suivies au cours du temps par différentes techniques analytiques, en particulier par la méthode dite du "head space" en chromatographie gazeuse.

Dans les différentes applications des mercaptoacides, et plus particulièrement dans leurs applications cosmétiques, l'odeur dégagée par les produits mis en oeuvre constitue une réelle gêne pour les utilisateurs. On a donc essayé de masquer l'odeur des mercaptoacides par des parfums, mais, en général, cette odeur est trop puissante pour pouvoir être masquée de façon satisfaisante.

On a également proposé, dans la demande de brevet japonais n° 82-136 280 publiée sous le n° 84/027 866, de désodoriser l'acide thioglycolique, pur ou en mélange avec de l'eau, par extraction par un hydrocarbure non aromatique en C₄-C₈. Mais on a constaté que, si ce procédé d'extraction permet d'extraire les composés malodorants et d'obtenir un acide désodorisé, l'effet de désodorisation obtenu n'est pas durable dans le temps car les composés malodorants se reforment rapidement et annulent le bénéfice obtenu par le traitement ; dans certains cas, l'odeur revient même à un niveau supérieur au niveau initial.

Par ailleurs, il est connu d'utiliser comme fluides d'extraction, des fluides supercritiques.Les fluides supercritiques sont des phases qui sont à une température et une pression supérieures à des valeurs critiques caractéristiques du composé considéré ; dans le domaine supercritique, il n'existe pas de courbe d'équilibre liquide/gaz séparant un domaine, où le composé serait gazeux, d'un domaine, où il serait liquide. Une extraction par un fluide supercritique présente de nombreux avantages ; ceci est particulièrement vrai quand on utilise le dioxyde de carbone : il n'y a pas contamination par un solvant résiduel ; la température critique de CO₂ est proche de la température ambiante, ce qui permet d'effectuer une extraction dans des conditions douces et dans un environnement non oxydant; CO₂ est un liquide non inflammable et non toxique. De tels procédés d'extraction sont, par exemple, décrits dans P. HUBERT et O. G. VITZHUM Angew. Chem. Int. Ed. Engl 17, 710-715 (1978); F. TEMELLI et al, Food Techn. 42 (6) 145-150 (1988); D. A. MOYLER "Supercritical fluids for extract preparation in Distilled Beverage Flavor - Recent developments" J. R. PIGOTT and A. PATERSON, Chichester Editions, England, Ellis HORWOOD Ltd (1989); J. W. KING, Cosmetic and Toiletries, 106 (8) 61-67 (1991). Dans tous ces procédés, on obtient un extrait gazeux contenant la substance recherchée et un résidu liquide d'extraction, qui peut être rejeté. D'autre part, comme indiqué dans l'article J. W. KING cité ci-dessus, les produits traités sont des composés non polaires, analogues à des lipides et très hydrophobes.

La présente invention concerne un procédé de désodorisation de mercaptoacides par extraction à l'aide d'un fluide supercritique ; un tel procédé permet d'obtenir un produit durablement désodorisé.

La présente invention a donc pour objet un procédé de désodorisation d'un mercaptoacide à l'aide d'un fluide d'extraction, ce procédé consistant à séparer et extraire par ledit fluide la majeure partie des produits de décomposition malodorants contenus dans le mercaptoacide soumis au traitement, caractérisé par le fait que le mercaptoacide a pour formule:

HS - A - COOH (I)

formule dans laquelle A représente
- le radical divalent :

   -(CH₂)ₙ-

   où n est un nombre entier compris entre 1 et 4 ;
- le radical divalent : 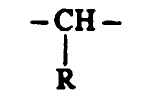 où R représente un radical alkyle, linéaire ou ramifié, en C₁-C₃ ; ou :
- le radical divalent : 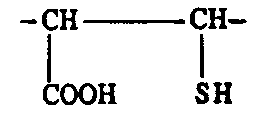 et que le fluide d'extraction est un fluide supercritique constitué d'un composé dont la température critique est comprise entre 0 et 135° C et la pression critique entre 10⁶ et 10⁷ pascals, le procédé étant mis en oeuvre en présence d'au moins un solvant polaire du mercaptoacide.

Le composé à l'état de fluide supercritique, que l'on utilise de préférence, est le dioxyde de carbone (CO₂).

Le mercaptoacide traité est, de préférence, l'acide thioglycolique ou l'acide thiolactique ; on peut cependant citer également l'acide 4-mercapto butyrique, l'acide 3-mercapto propionique et l'acide di-mercapto-succinique.

Le solvant polaire est, de préférence, choisi dans le groupe formé par l'eau, les monoalcools linéaires ou ramifiés en C₁-C₅, les diols en C₂-C₆, les polyols en C₃-C₆ et leurs mélanges.

Le monoalcool peut être choisi dans le groupe formé par le méthanol, l'éthanol, le 1-propanol, le 2-propanol, le 1-butanol, le 2-butanol, le 2-méthyl propanol et le 1-pentanol.

Le diol est avantageusement choisi dans le groupe formé par l'éthylèneglycol, le 1,2-propanediol, le 1,3-propanediol, le 1,3-butanediol, le 2,3-butanediol, le 1,5-pentanediol, le 1,6-hexanediol.

Le polyol peut avantageusement être le glycérol.

L'eau est le solvant préféré car, dans la plupart des applications du mercaptoacide traité, son élimination ultérieure n'est pas nécessaire.

La présence d'un solvant pour effectuer l'extraction est nécessaire. En effet, des essais ont montré que les mercaptoacides de formule (I), en particulier l'acide thioglycolique, étaient miscibles en toutes proportions avec un fluide supercritique, en particulier avec le CO₂ supercritique, le mélange ne présentant aucune discontinuité : il ne se forme donc qu'une seule phase et il n'est pas possible d'effectuer une extraction. Cela conduisait logiquement l'homme de métier à écarter tout traitement par fluide supercritique pour la désodorisation des mercaptoacides. Selon la présente invention, on a constaté qu'en introduisant dans le milieu d'extraction un solvant polaire, on provoque une démixtion avec formation d'au moins deux phases ; les composés malodorants du mercaptoacide sont extraits par le fluide supercritique et, quand on détend le milieu d'extraction, ils sont entraînés avec le fluide d'extraction ; le résidu est constitué par une solution de mercaptoacide dans le solvant polaire utilisé.

Le procédé selon l'invention permet donc d'obtenir un mercaptoacide, qui est désodorisé et qui a son odeur légèrement piquante propre. Le fait qu'il soit désodorisé ne veut pas obligatoirement dire qu'il soit pur ; en effet, il pourrait contenir des impuretés n'ayant pas d'odeur. De plus, de façon tout à fait inattendue, on a constaté qu'après au moins deux mois de conservation de la solution de mercaptoacide à température ambiante à l'abri de l'oxygène de l'air, il n'est pas possible de détecter à l'odorat la présence des composés malodorants. Par conséquent, ceux-ci ne se sont pas reformés ou ne sont reformés que dans des proportions très faibles, puisque non perceptibles au nez.

Le solvant est utilisé en quantités comprises entre 1 et 99 % en poids par rapport au mercaptoacide. Il peut être ajouté aussi bien au mercaptoacide qu'au fluide supercritique utilisé. La quantité de solvant, qui peut être introduite, est limitée par la solubilité maximum dudit solvant dans le mercaptoacide et dans le fluide d'extraction.

Lorsque le fluide supercritique est du dioxyde de carbone, on utilise, de préférence, une solution aqueuse de mercaptoacide ayant une concentration comprise entre 0,1 mole par litre et 14 moles par litre, de préférence entre 1 mole par litre et 10 moles par litre.

Selon l'invention, on peut améliorer l'efficacité de l'extraction vis-à-vis de la quantité de fluide supercritique mise en oeuvre en effectuant ladite extraction en présence d'un gaz inerte tel que l'hélium, l'azote ou l'argon ; ce gaz inerte peut être introduit dans le milieu d'extraction séparément ou en mélange avec le fluide supercritique.

Selon l'invention, l'extraction peut être effectuée aussi bien en continu qu'en semi-continu ou en discontinu.

Lorsqu'on opère en discontinu, on introduit, dans le réacteur, le mercaptoacide, le solvant polaire, le fluide d'extraction et, éventuellement, le gaz inerte, on fait en sorte que les conditions de pression et de température critiques soient obtenues dans le réacteur fermé. On laisse en contact, de préférence sous agitation, pendant un cycle de durée comprise entre 5 et 30 minutes, au cours duquel le fluide supercritique se charge en composés maladorants ; on enlève ensuite le fluide d'extraction par décompression. On effectue éventuellement un ou plusieurs cycles dans les mêmes conditions et on recueille une solution de mercaptoacide désodorisé.

Lorsqu'on opère en semi-continu, on introduit le mercaptoacide et le solvant polaire, on remplit le réacteur de fluide d'extraction et l'on amène la pression et la température dans le réacteur à des valeurs supérieures à la pression et la température critiques. On assure ensuite un débit de lavage par le fluide supercritique pendant une durée déterminée, débit qui peut être constant ou variable, ce débit étant fonction de l'installation. Dans le cas de CO₂ supercritique, il est généralement compris entre 0,5 et 3 kg/heure ; il est cependant préférable de rapporter ce débit à la quantité du mélange (mercaptoacide-solvant). Dans le cas de l'acide thioglycolique en solution aqueuse et de CO₂ supercritique, le débit de dioxyde de carbone est compris entre 1 et 30 kg/heure et par kg de mélange (acide thioglycolique-solvant). Pour finir, on arrête le débit de fluide supercritique et on recueille la solution de mercaptoacide.

Lorsqu'on opère en continu, on réalise l'extraction du mélange (mercaptoacide/solvant) par un fluide supercritique à courants parallèles ou à contre-courant sur une colonne de séparation. On assure à la fois un débit continu du mélange à extraire et du fluide supercritique. Le rapport des deux débits est fixé en fonction de l'efficacité de séparation, que l'on désire.

Après séparation du dioxyde de carbone, le résidu liquide désodorisé recueilli est constitué par le mercaptoacide et la majeure partie du solvant ; il contient éventuellement de faibles quantités de fluide d'extraction, par exemple de CO₂. La solution de mercaptoacide désodorisé obtenue est, de préférence, soigneusement conditionnée à l'abri de l'oxygène de l'air dans un conteneur connu pour sa compatibilité chimique avec le mercaptoacide, éventuellement sans éliminer le fluide d'extraction résiduel, par exemple le CO₂.

Le solvant peut cependant être éliminé par tout procédé connu avant stockage, en particulier lorsque le solvant est autre que l'eau.

Quel que soit le procédé d'extraction utilisé, le fluide d'extraction peut être recyclé après avoir été débarrassé des composés extraits par purification selon des méthodes connues telles que le passage sur un lit de charbon actif.

La présente invention a également pour objet le produit désodorisé obtenu par le procédé de la présente invention.

Les solutions de mercaptoacide de formule (I) désodorisé peuvent être utilisées dans toutes les applications industrielles connues pour ces produits. Le mercaptoacide est généralement associé avec d'autres ingrédients dans une composition dont la formulation varie selon l'application. L'efficacité de la désodorisation permet de les utiliser, sans donner d'odeur désagréable, dans toute composition qui ne doit pas avoir d'odeur désagréable et dont les autres ingrédients ne possèdent pas d'odeur désagréable. On peut mentionner, à titre d'exemple, la possibilité d'association avec des thiols tels que la cystéine ou la cystéamine qui ne présentent pas d'odeur désagréable.

Lorsque la composition utilisée contient des ingrédients susceptibles de faire perdre le bénéfice de la désodorisation en provoquant la formation de composés malodorants, il peut être avantageux de conditionner séparément la solution de mercaptoacide obtenue et de préparer la quantité de composition nécessaire à l'application au dernier moment.

Les exemples donnés ci-après, à titre illustratif et non limitatif, permettront de mieux comprendre l'invention.

Les exemples 1 à 6 illustrent le procédé de désodorisation selon l'invention.

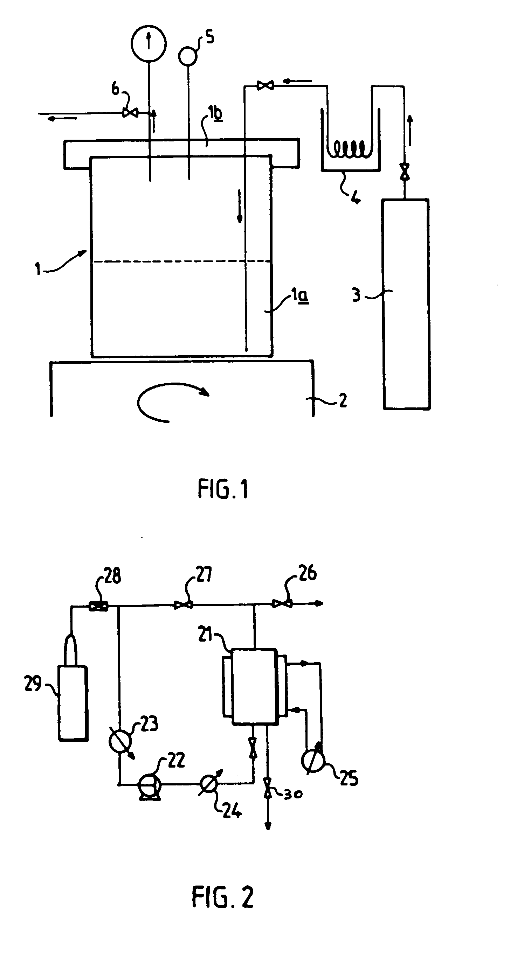

Les essais des exemples 1 à 3 ont été effectués dans un réacteur d'extraction en discontinu schématisé sur la figure 1. L'installation comporte un réacteur 1 constitué d'une enveloppe d'acier 1a résistant à la pression et d'un couvercle 1b. Le réacteur 1 est muni d'un système d'agitation et de chauffage schématisé en 2. Le fluide d'extraction est stocké sous pression dans un réservoir 3 ; il est envoyé dans le réacteur 1 par l'intermédiaire d'un condenseur 4, pour assurer un remplissage du réacteur avec un fluide d'extraction à l'état liquide. Le réacteur 1 est muni d'une soupape de sécurité 5. Il est relié par l'intermédiaire d'une vanne 6 à un restricteur (non représenté) qui assure la décompression lente du réacteur.

### EXEMPLE 1

On introduit dans le réacteur 1, qui a une capacité de 100 ml, 35 ml d'une solution aqueuse d'acide thioglycolique à 92 g/l (1M). Le réacteur est alors rempli de dioxyde de carbone liquide à 8° C à partir d'un réservoir 3 constitué par une bouteille de CO₂ à 60 bars et 20° C. Après avoir isolé le réacteur 1, on porte son contenu à 60° C. La pression observée est de 145 x 10⁵ pascals. On laisse le contenu du réacteur 1 sous agitation pendant 20 minutes. On ouvre alors la vanne 6, qui débite sur un restricteur constitué par une tubulure de 30 cm de long et 50 µm de diamètre intérieur. Lorsque la pression atteint 50 bars, l'appareil est refroidi à 8° C, purgé et l'on effectue un second cycle identique à celui qui vient d'être décrit.

On soutire la solution aqueuse d'acide thioglycolique obtenue qui ne possède plus l'odeur nauséabonde initiale du composé, mais une légère odeur piquante.

### EXEMPLE 2

On introduit, dans le réacteur 1, 50 ml de solution aqueuse d'acide thioglycolique à 368 g/l (4M). On remplit le réacteur de CO₂ liquide à 60 bars et 15° C et on chauffe à 41° C jusqu'à ce que la pression atteigne 110 x 10⁵ pascals. Le temps de contact est de 15 minutes. On effectue deux cycles successifs.

La solution d'acide thioglycolique obtenue n'a plus d'odeur nauséabonde.

### EXEMPLE 3

On a effectué, comme dans l'exemple 1, deux cycles d'extraction sur 50 ml d'une solution aqueuse d'acide thioglycolique à 92 g/l (1M). Le dioxyde de carbone liquide est introduit à 60 bars et 20° C puis le contenu du réacteur est porté à 35° C, la pression étant ajustée à 130 x 10⁵ pascals à l'aide d'hélium gazeux.

La solution aqueuse d'acide thioglycolique recueillie n'a plus d'odeur nauséabonde.

Les exemples 4 à 6 donnés ci-après ont été effectués en semi-continu dans l'installation schématisée sur la figure 2. Cette installation comporte un autoclave 21 alimenté par une pompe 22 de circulation et de mise en pression du fluide d'extraction. En amont de la pompe 22 est disposé un condenseur 23 pour liquéfier le fluide d'extraction. En aval de la pompe 22 est prévu un échangeur de chaleur 24 pour amener le fluide d'extraction dans les conditions de température de l'extraction. Un échangeur de chaleur 25 permet de réguler la température de l'autoclave. A la sortie de l'autoclave 21, le flux de matière est partiellement soutiré par la vanne micrométrique 26 et partiellement recyclé par la vanne micrométrique 27, le réglage relatif de ces deux vannes assurant la régulation de la pression d'extraction. L'alimentation en fluide d'extraction est assurée, à travers un clapet anti-retour 28, à partir d'un container 29 en direction du condenseur 23. La vidange de l'autoclave 21 s'effectue par la vanne 30.

### EXEMPLE 4

100 ml d'une solution aqueuse d'acide thioglycolique à 184 g/l (2M) sont chargés dans l'autoclave 21. Après mise sous pression de CO₂ liquide dans la totalité de l'installation, on assure à l'aide de la pompe 22 un débit de CO₂ supercritique dans les conditions d'extraction suivantes :
- pression : 250 x 10⁵ pascals
- température : 40° C
- débit de circulation : 2,4 kg/h
Le temps d'extraction est fixé à 20 minutes. Après décompression et vidange de l'autoclave 21, la solution d'acide thioglycolique ne présente plus l'odeur désagréable initiale.

### EXEMPLE 5

100 ml d'une solution hydroalcoolique d'acide thioglycolique à 184 g/l (2M) sont chargés dans l'autoclave 21. Le solvant est composé de 2-propanol en solution dans l'eau, au titre massique de 10 %. On assure à l'aide de la pompe 22 un débit de CO₂ supercritique dans les conditions d'extraction suivantes :
- pression : 200 x 10⁵ pascals
- température : 35° C
- débit de circulation : 2,1 kg/h
Le temps d'extraction est fixé à 20 minutes. Après décompression et vidange, la solution hydralcoolique ne présente plus l'odeur désagréable initiale.

### EXEMPLE 6

100 ml d'une solution d'acide thioglycolique à 184 g/l (2M) sont chargés dans l'autoclave 21. Le solvant est composé de glycérine en solution dans l'eau au titre massique de 3 %. Les conditions d'extraction sont :
- pression : 200 x 10⁵ pascals
- température : 35° C
- débit de circulation : 2,1 kg/h
Un temps d'extraction de 20 minutes assure un résultat olfactif très positif: la solution ne présente plus l'odeur désagréable initiale.

Les exemples 7 à 13 concernent des applications de solutions d'acide mercaptoacétique selon l'invention.

### EXEMPLE 7

On prépare :

### A - Composition réductrice :

Cette préparation se fait à partir de deux solutions stockées dans des flacons différents.
Solution (a) :
   - Solution aqueuse d'acide thioglycolique désodorisé à 36 % de matière active 25 g
Solution (b) :
   - Oxyde de laurylamine commercialisé sous la dénomination "AROMOX DMMCD/W" par la société "AKZO" 2 g
   - Acide éthylènediamine tétraacétique 0,15 g
   - Monoéthanolamine qsp pH = 9,0
   - Eau déminéralisée qsp 75 g

Après mélange de la solution (a) avec la solution (b), on obtient une composition réductrice ayant un pH de 9,0.

On applique cette composition réductrice sur des cheveux mouillés, préalablement enroulés sur des bigoudis, puis on recouvre les cheveux d'un bonnet en matière plastique et on laisse agir pendant 15 minutes à température ambiante. On rince ensuite abondamment les cheveux à l'eau et on applique alors la composition oxydante définie en B.

### B - Une composition oxydante

- Eau oxygénée 2 g
- Stannate de sodium 0,015 g
- Lauryl sulfate d'ammonium 1,4 g
- Hydrolysat de protéines 0,6 g
- Acide citrique 0,5 g
- Parfum qs
- Eau déminéralisée qsp 100 g

On laisse agir la composition oxydante pendant 5 minutes puis on déroule les cheveux et laisse encore agir 3 minutes. On rince enfin abondamment à l'eau.

Après séchage sous casque, on constate que les cheveux présentent de belles boucles avec un bon degré de frisure.

On a constaté, à l'odorat, qu'au cours de l'opération, il ne se développe pas d'odeur nauséabonde.

### EXEMPLE 8

En utilisant le même mode opératoire que celui décrit dans l'exemple 7, on a procédé à une déformation permanente des cheveux à l'aide des compositions réductrice et oxydante suivantes :

### A - Composition réductrice

- Solution (a)
   - Solution aqueuse d'acide thioglycolique désodorisé à 18 % de matière active 50 g
Solution (b) :
   - Oxyde de laurylamine commercialisé sous la dénomination "AROMOX DMMCD/W" par la société "AKZO" 0,9 g
   - Sel pentasodique de l'acide diéthylène triamine pentaacétique 0,15 g
   - Parfum qs
   - Ammoniaque (20 % dans l'eau) qsp pH = 7,8
   - Eau déminéralisée qsp 50 g

On obtient par mélange une composition ayant un pH de 7,8.

### B - Composition oxydante

On utilise la même composition oxydante que celle décrite dans l'exemple 7.

On a constaté, à l'odorat, qu'au cours de l'opération, il ne se développe pas d'odeur nauséabonde.

### EXEMPLE 9

En utilisant le même mode opératoire que celui décrit dans l'exemple 7, on a procédé à une déformation permanente des cheveux, à l'aide des compositions réductrice et oxydante suivantes :

### A - Composition réductrice

- Solution (a)
   - Solution aqueuse d'acide thiolactique désodorisé à 22 % de matière active 50 g
Solution (b) :
   - Oxyde de laurylamine commercialisé sous la dénomination "AROMOX DMMCD/W" par la société "AKZO" 0,9 g
   - Parfum qs
   - Ammoniaque (20 % dans l'eau) qsp pH = 8,2
   - Eau déminéralisée qsp 50 g

On obtient par mélange une composition ayant un pH de 8,2.

### B - Composition oxydante

On utilise la même composition oxydante que celle décrite à l'exemple 7.

On a constaté, à l'odorat, qu'au cours de l'opération, il ne se développe pas d'odeur nauséabonde.

### EXEMPLE 10

En utilisant le même mode opératoire que celui décrit dans l'exemple 7, on a procédé à une déformation permanente des cheveux, à l'aide des compositions réductrice et oxydante suivantes :

### A - Composition réductrice

- Solution (a)
   - Solution aqueuse d'acide thioglycolique désodorisé à 18 % de matière active 50 g
Solution (b) :
   - Cystéine 4,5 g
   - Ester stéarique polyoxyéthyléné avec 8 moles d'oxyde d'éthylène commercialisé sous la dénomination "MYRJ 45" par la société "ICI" 0,85 g
   - Conservateur 0,35 g
   - Parfum qs
   - Ammoniaque qsp pH = 8,8
   - Eau déminéralisée qsp 50 g

On obtient par mélange une composition ayant un pH de 8,8.

### B - Composition oxydante

- Eau oxygénée (20 volumes) 40 g
- Acide citrique qsp pH = 3,2
- Eau déminéralisée qsp 100 g

On a constaté, à l'odorat, qu'au cours de l'opération, il ne se développe pas d'odeur nauséabonde.

### EXEMPLE 11

En utilisant le même mode opératoire que celui décrit dans l'exemple 7, on a procédé à une déformation permanente des cheveux, à l'aide des compositions réductrice et oxydante suivantes :

### A - Composition réductrice

- Solution (a)
   - Solution aqueuse d'acide thioglycolique désodorisé à 18 % de matière active 40 g
Solution (b) :
   - Chlorhydrate de cystéamine 6,0 g
   - Oxyde de laurylamine commercialisé sous la dénomination "AROMOX DMMCD/W" par la société "AKZO" 2 g
   - Conservateur 0,15 g
   - Parfum qs
   - Monoéthanolamine qsp pH = 7,8
   - Eau déminéralisée qsp 60 g

On obtient par mélange une composition ayant un pH de 7,8.

### B - Composition oxydante

- Bromate de sodium 8 g
- Triéthanolamine qsp pH = 8
- Phosphate monosodique hydraté (12H₂0) 0,3 g
- Phosphate trisodique hydraté 0,5 g
- Cocoamidopropylbétaïne commercialisé sous la dénomination "TEGOBETAINE HS" par la société "GOLDSCHMIDT" 1 g
- Parfum qs
- Eau déminéralisée qsp 100 g

On a constaté, à l'odorat, qu'au cours de l'opération, il ne se développe pas d'odeur nauséabonde.

### EXEMPLE 12

On a utilisé :

### A - Une composition réductrice

stockée dans un seul flacon et ayant la composition suivante:
- Solution aqueuse d'acide thioglycolique désodorisé à 36 % de matière active 25 g
- Oxyde de laurylamine commercialisé sous la dénomination "AROMOX DMMCD/W" par la société "AKZO" 2 g
- Acide éthylènediamine tétraacétique 0,15 g
- Monoéthanolamine qsp pH = 9
- Eau déminéralisée qsp 100 g

On applique cette composition réductrice sur des cheveux mouillés, préalablement enroulés sur des bigoudis, puis on recouvre les cheveux d'un bonnet en matière plastique et on laisse ensuite agir pendant 15 minutes à température ambiante. On rince alors abondamment les cheveux à l'eau et on applique la composition oxydante suivante:

### B - Composition oxydante

- Eau oxygénée 2 g
- Stannate de sodium 0,015 g
- Lauryl sulfate d'ammonium 1,4 g
- Hydrolysat de protéines 0,6 g
- Acide citrique 0,5 g
- Parfum qs
- Eau déminéralisée qsp 100 g

On a constaté, à l'odorat, qu'au cours de l'opération, il ne se développe pas d'odeur nauséabonde.

### EXEMPLE 13

On prépare :

### A - Une composition réductrice de défrisage à partir de deux solutions conservées dans des flacons différents :

- Solution (a)
   - Solution aqueuse d'acide thioglycolique désodorisé à 18% de matière active 50 g
Solution (b) :
   - Acide polyacrylique commercialisé sous la dénomination "CARBOPOL 934" par la société "GOODRICH" 8 g
   - Ammoniaque (20 % dans l'eau) qsp pH = 8,2
   - Parfum qs
   - Eau déminéralisée qsp 50 g

Après mélange de la solution (a) avec la solution (b), on obtient une composition réductrice de défrisage ayant un pH de 8,2.

On applique cette composition sur des cheveux mouillés, initialement bouclés, et on lisse les cheveux, de façon à les rendre raides, à l'aide d'un peigne. Après un temps de pose de 10 minutes, on rince abondamment les cheveux à l'eau et on applique la composition oxydante suivante:

### B - Composition oxydante

- Eau oxygénée 2 g
- Stannate de sodium 0,015 g
- Lauryl sulfate d'ammonium 1,4 g
- Hydrolysat de protéines 0,6 g
- Acide citrique 0,5 g
- Parfum qs
- Eau déminéralisée qsp 100 g

On laisse agir la composition oxydante pendant 5 minutes puis on rince abondamment à l'eau.

On a constaté, à l'odorat, qu'au cours de l'opération, il ne se développe pas d'odeur nauséabonde.

### EXEMPLE 14 : Composition dépilatoire

On prépare :
- Solution (a)
   - Solution aqueuse d'acide thioglycolique désodorisé à 18 % de matière active 20 g
Solution (b):
   - Urée 5 g
   - Carbonate de calcium 1 g
   - Alcool cétylstéarylique et cétylstéarylique oxyéthyléné (mélange 80/20) 10 g
   - Parfum qs
   - Eau déminéralisée qsp 80 g

On mélange la solution (a) avec la solution (b) au moment de l'emploi et on ajuste le pH à 11,2 par addition d'hydroxyde de calcium.

On applique cette composition sur la peau et on laisse agir 10 minutes puis on rince abondamment à l'eau.

On a constaté, à l'odorat, qu'au cours de l'opération, il ne se développe pas d'odeur nauséabonde.

## Revendications

1. Procédé de désodorisation d'un mercaptoacide à l'aide d'un fluide d'extraction consistant à séparer et extraire par ledit fluide la majeure partie des produits de décomposition malodorants contenus dans ledit mercaptoacide, caractérisé par le fait que le mercaptoacide a pour formule :
HS - A - COOH (I)
formule dans laquelle A représente
- le radical divalent :
-(CH₂)ₙ-
où n est un nombre entier compris entre 1 et 4 ;
- le radical divalent : 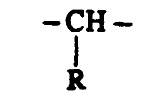 où R représente un radical alkyle, linéaire ou ramifié, en C₁-C₃ ; ou :
- le radical divalent : 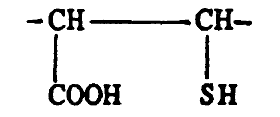 et que le fluide d'extraction est un fluide supercritique constitué d'un composé dont la température critique est comprise entre 0 et 135° C et la pression critique entre 10⁶ et 10⁷ pascals, le procédé étant mis en oeuvre en présence d'au moins un solvant polaire du mercaptoacide.

2. Procédé selon la revendication 1, caractérisé par le fait que le composé à l'état de fluide supercritique utilisé est le dioxyde de carbone.

3. Procédé selon l'une des revendications 1 ou 2, caractérisé par le fait que le mercaptoacide est l'acide thioglycolique ou l'acide thiolactique.

4. Procédé selon l'une des revendications 1 à 3, caractérisé par le fait que le solvant polaire est choisi dans le groupe formé par l'eau, les monoalcools linéaires ou ramifiés en C₁-C₅, les diols en C₂-C₆, les polyols en C₃-C₆ et leurs mélanges.

5. Procédé selon la revendication 4, caractérisé par le fait que le monoalcool est choisi dans le groupe formé par le méthanol, l'éthanol, le 1-propanol, le 2-propanol, le 1-butanol, le 2-butanol, le 2-méthyl propanol et le 1-pentanol.

6. Procédé selon la revendication 4, caractérisé par le fait que le diol est choisi dans le groupe formé par l'éthylène glycol, le 1,2-propanediol, le 1,3-propanediol, le 1,3-butanediol, le 2,3-butanediol, le 1,5-pentanediol et le 1,6-hexanediol.

7. Procédé selon la revendication 4, caractérisé par le fait que le polyol est le glycérol.

8. Procédé selon l'une des revendications 1 à 5, caractérisé par le fait que le solvant polaire est utilisé en une quantité comprise entre 1 et 99 % en poids par rapport au mercaptoacide.

9. Procédé selon la revendication 2, caractérisé par le fait qu'on utilise une solution aqueuse de mercaptoacide ayant une concentration comprise entre 0,1 mole par litre et 14 moles par litre.

10. Procédé selon l'une des revendications 1 à 9, caractérisé par le fait qu'on effectue l'extraction en présence d'un composé gazeux inerte.

11. Procédé selon la revendication 10, caractérisé par le fait que le composé gazeux inerte est choisi dans le groupe formé par l'hélium, l'azote et l'argon.

12. Procédé selon l'une des revendications 1 à 11 dans lequel on opère en discontinu, caractérisé par le fait qu'on introduit dans le réacteur, le mercaptoacide, le solvant polaire, le fluide d'extraction et, éventuellement, le gaz inerte, on amène les conditions de pression et de température dans le réacteur fermé à être supercritiques, on laisse en contact les produits placés dans le réacteur pendant 5 à 30 minutes, on enlève le fluide d'extraction par décompression, on effectue éventuellement un ou plusieurs autres cycles dans les mêmes conditions, et on recueille une solution de mercaptoacide désodorisé.

13. Procédé selon l'une des revendications 1 à 11 dans lequel on opère en semi-continu, caractérisé par le fait qu'on introduit le mercaptoacide et le solvant polaire dans le réacteur, on remplit le réacteur de fluide d'extraction et on amène la pression et la température dans le réacteur à dépasser la pression et la température critiques, on assure ensuite un débit de lavage par le fluide supercritique pendant un cycle de durée déterminée, on arrête le débit, on assure la décompression du réacteur et on recueille le mercaptoacide désodorisé.

14. Procédé selon les revendications 2 et 13 prises simultanément, caractérisé par le fait que le fluide d'extraction est le dioxyde de carbone et le débit est compris entre 0,5 et 3 kg/heure.

15. Procédé selon la revendication 14, dans lequel le mercaptoacide est l'acide thioglycolique, caractérisé par le fait que le débit de CO₂ est compris entre 1 et 30 kg/heure par kg de mélange (acide thioglycolique-solvant polaire).

16. Procédé selon l'une des revendications 1 à 11, caractérisé par le fait qu'on opère en continu dans une colonne de séparation.

17. Procédé selon l'une des revendications 12 à 15, caractérisé par le fait qu'on débarrasse le fluide d'extraction des impuretés malodorantes et qu'on le recycle.

## Patentansprüche

1. verfahren zur Desodorierung einer Mercaptosäure mit Hilfe einer Extraktionsflüssiqkeit, durch Abtrennung und Extraktion des größten Teils der in der Mercaptosäure enthaltenen übelriechenden Zersetzungssprodukte mittels dieser Flüssigkeit, dadurch gekennzeichnet, daß die Mercaptosäure folgende Formel besitzt:
HS - A - COOH (I)
worin A für
- den divalenten Rest:
- (CH₂)ₙ -
worin n für eine ganze Zahl von 1 bis 4 steht;
- den divalenten Rest: 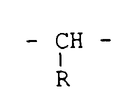 worin R für einen linearen oder verzweigten C₁-C₃-Alkylrest steht; oder:
- den divalenten Rest: 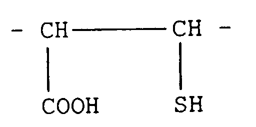 steht;
und daß die Extraktionsflüssigkeit eine superkritische Flüssigkeit ist, bestehend aus einer Verbindung, deren kritische Temperatur im Bereich von 0 bis 135°C und deren kritischer Druck im Bereich von 10⁶ bis 10⁷ Pascal liegt, und daß das Verfahren in Gegenwart wenigstens eines polaren Lösungsmittels für die Mercaptosäure durchgeführt wird.

2. Verfahren gemäß Anspruch 1, dadurch gekennzeichnet, daß es sich bei der verwendeten Verbindung im Zustand einer superkritischen Flüssigkeit um Kohlendioxid handelt.

3. Verfahren gemäß einem der Ansprüche 1 oder 2, dadurch gekennzeichnet, daß es sich bei der Mercaptosäure um Thioglycolsäure oder um Thiomilchsäure handelt.

4. Verfahren gemäß einem der Ansprüche 1 bis 3, dadurch gekennzeichnet, daß das polare Lösungsmittel ausgewählt ist unter Wasser, linearen oder verzweigten C₁-C₅-Monoalkoholen, C₂-C₆-Diolen, C₃-C₆-Polyolen und Gemischen davon.

5. Verfahren gemäß Anspruch 4, dadurch gekennzeichnet, daß der Monoalkohol ausgewählt ist unter Methanol, Ethanol, 1-Propanol, 2-Propanol, 1-Butanol, 2-Butanol, 2-Methylpropanol und 1-Pentanol.

6. Verfahren gemäß Anspruch 4, dadurch gekennzeichnet, daß das Diol ausgewählt ist unter Ethylenglycol, 1,2-Propandiol, 1,3-Propandiol, 1,3-Butandiol, 2,3-Butandiol, 1,5-Pentandiol und 1,6-Hexandiol.

7. Verfahren gemäß Anspruch 4, dadurch gekennzeichnet, daß es sich bei dem Polyol um Glycerin handelt.

8. Verfahren gemäß einem der Ansprüche 1 bis 5, dadurch gekennzeichnet, daß das polare Lösungsmittel in einer Menge im Bereich von 1 bis 99 Gew.-%, bezogen auf die Mercaptosäure, verwendet wird.

9. Verfahren gemäß Anspruch 2, dadurch gekennzeichnet, daß man eine wässrige Lösung der Mercaptosäure mit einer Konzentration im Bereich von 0,1 mol/l bis 14 mol/l verwendet.

10. Verfahren gemäß einem der Ansprüche 1 bis 9, dadurch gekennzeichnet, daß man die Extraktion in Gegenwart eines Inertgases durchführt.

11. Verfahren gemäß Anspruch 10, dadurch gekennzeichnet, daß das Inertgas ausgewählt ist unter Helium, Stickstoff und Argon.

12. Verfahren gemäß einem der Ansprüche 1 bis 11, wobei man diskontinuierlich arbeitet, dadurch gekennzeichnet, daß man in den Reaktor die Mercaptosäure, das polare Lösungsmittel, die Extraktionsflüssigkeit und gegebenenfalls das Inertgas gibt, im geschlossenen Reaktor Druck und Temperatur im superkritischen Bereich einstellt, die im Reaktor vorgelegten Stoffe für 5 bis 30 min in Kontakt beläßt, die Extraktionsflüssigkeit durch Dekompression entfernt, gegebenenfalls ein- oder mehrmals weitere Zyklen unter den gleichen Bedingungen durchführt und eine desodorierte Lösung der Mercaptosäure gewinnt.

13. Verfahren gemäß einem der Ansprüche 1 bis 11, wobei man halb-kontinuierlich arbeitet, dadurch gekennzeichnet, daß man die Mercaptosäure und das polare Lösungsmittel in den Reaktor gibt, den Reaktor mit der Extraktionsflussigkeit befüllt und Druck und Temperatur im Reaktor so einstellt, daß sie den kritischen Druck und die kritische Temperatur überschreiten, anschließend für eine bestimmte Dauer mit der superkritischen Flüssigkeit durchspült, den Durchfluß abstellt, den Reaktor dekomprimiert und die desodorierte Mercaptosäure gewinnt.

14. Verfahren gemäß einer Kombination der Ansprüche 2 und 13, dadurch gekennzeichnet, daß es sich bei der Extraktionsflüssigkeit um Kohlendioxid handelt und daß der Durchfluß im Bereich von 0,5 bis 3 kg/Stunde liegt.

15. Verfahren gemäß Anspruch 14, wobei es sich bei der Mercaptosäure um Thioglycolsäure handelt, dadurch gekennzeichnet, daß der Durchfluß an CO₂ im Bereich von 1 bis 30 kg/Stunde pro kg Mischung (Thioglycolsäure, polares Lösungsmittel) liegt.

16. Verfahren gemäß einem der Ansprüche 1 bis 11, dadurch gekennzeichnet, daß man kontinuierlich in einer Trennkolonne arbeitet.

17. Verfahren gemäß einem der Ansprüche 12 bis 15, dadurch gekennzeichnet, daß man die Extraktionsflüssigkeit von den übelriechenden Verunreinigungen befreit und wieder zurückführt.

## Claims

1. Process for deodorising a mercapto acid using an extraction fluid, consisting in separating and extracting with the said fluid the greater part of the malodorous decomposition products contained in the said mercapto acid, characterised in that the mercapto acid is of the formula:
HS - A - COOH (I)
in which formula A represents
- the divalent radical:
-(CH₂)ₙ-
where n is an integer between 1 and 4;
- the divalent radical: 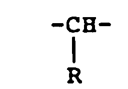 where R represents a linear or branched C₁-C₃ alkyl radical;
or
- the divalent radical: 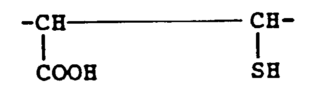 and in that the extraction fluid is a supercritical fluid consisting of a compound whose critical temperature is between 0 and 135°C and critical pressure between 10⁶ and 10⁷ pascals, the process being carried out in the presence of at least one polar solvent for the mercapto acid.

2. Process according to Claim 1, characterised in that the compound in the state of supercritical fluid which is used is carbon dioxide.

3. Process according to one of Claims 1 or 2, characterised in that the mercapto acid is thioglycolic acid or thiolactic acid.

4. Process according to one of Claims 1 to 3, characterised in that the polar solvent is chosen from the group composed of water, linear or branched C₁-C₅ monohydric alcohols, C₂-C₆-diols, C₃-C₆ polyols and mixtures thereof.

5. Process according to Claim 4,
characterised in that the monohydric alcohol is chosen from the group composed of methanol, ethanol, 1-propanol, 2-propanol, 1-butanol, 2-butanol, 2-methylpropanol and 1-pentanol.

6. Process according to Claim 4,
characterised in that the diol is chosen from the group composed of ethylene glycol, 1,2-propanediol, 1,3-propanediol, 1,3-butanediol, 2,3-butanediol, 1,5-pentanediol and 1,6-hexanediol.

7. Process according to Claim 4,
characterised in that the polyol is glycerol.

8. Process according to one of Claims 1 to 5, characterised in that the polar solvent is used in an amount of between 1 and 99 % by weight relative to the mercapto acid.

9. Process according to Claim 2,
characterised in that an aqueous solution of mercapto acid having a concentration of between 0.1 mol per litre and 14 mol per litre is used.

10. Process according to one of Claims 1 to 9, characterised in that the extraction is performed in the presence of an inert gaseous compound.

11. Process according to Claim 10, characterised in that the inert gaseous compound is chosen from the group composed of helium, nitrogen and argon.

12. Process according to one of Claims 1 to 11, in which the procedure is carried out in discontinuous fashion, characterised in that the mercapto acid, the polar solvent, the extraction fluid and, where appropriate, the inert gas are introduced into the reactor, the pressure and temperature conditions in the closed reactor are taken to the supercritical state, the products placed in the reactor are left in contact for 5 to 30 minutes, the extraction fluid is removed by decompression, one or more further cycles are performed, where appropriate, under the same conditions, and a solution of deodorised mercapto acid is collected.

13. Process according to Claims 1 to 11, in which the procedure is carried out in semi-continuous fashion, characterised in that the mercapto acid and the polar solvent are introduced into the reactor, the reactor is filled with extraction fluid and the pressure and temperature in the reactor are taken to values exceeding the critical pressure and temperature, a washing flow with supercritical fluid is then established for a cycle lasting for a specified period, the flow is stopped, decompression of the reactor is effected and the deodorised mercapto acid is collected.

14. Process according to Claims 2 and 13 taken simultaneously, characterised in that the extraction fluid is carbon dioxide and the flow is at between 0.5 and 3 kg/hour.

15. Process according to Claim 14, in which the mercapto acid is thioglycolic acid, characterised in that the flow of CO₂ is at between 1 and 30 kg/hour per kg of (thioglycolic acid/polar solvent) mixture.

16. Process according to one of Claims 1 to 11, characterised in that the procedure is carried out in continuous fashion in a separating column.

17. Process according to one of Claims 12 to 15, characterised in that the extraction fluid is relieved of the malodorous impurities and in that it is recycled.
